# EUROPEAN PATENT SPECIFICATION

(11) **EP 4 063 983 B1**
(45) Date of publication and mention of the grant of the patent: **19.02.2025**
(21) Application number: 22158839.5
(22) Date of filing: 25.02.2022
(51) Int. Cl.: G05B 23/02, G11B 20/00

(54) **FIELD DEVICE, MEASUREMENT METHOD, AND NON-TRANSITORY COMPUTER READABLE MEDIUM**
FELDVORRICHTUNG, MESSVERFAHREN UND NICHTFLÜCHTIGES COMPUTERLESBARES MEDIUM
DISPOSITIF DE CHAMP, PROCÉDÉ DE MESURE ET SUPPORT NON TRANSITOIRE LISIBLE PAR ORDINATEUR

(30) Priority: 23.03.2021 JP 2021049140
(43) Date of publication of application: 28.09.2022
(73) Proprietor: Yokogawa Electric Corporation, Musashino-shi, Tokyo 180-8750 (JP)
(72) Inventor: EBISUMOTO, Shunsuke, Musashino-shi, Tokyo 180-8750 (JP)
(74) Representative: Hoffmann Eitle

(56) References cited:
- EP-A1- 3 309 682
- US-A1- 2004 030 524
- US-A1- 2013 097 128
- US-A1- 2018 314 243
- KIARIE JAMES: "How to Rotate and Compress Log Files in Linux with Logrotate", 26 May 2020 (2020-05-26), pages 1 - 20, XP055948731, Retrieved from the Internet <URL:https://www.linuxtechi.com/manage-linux-log-files-using-logrotate/> [retrieved on 20220803]

## Description

### TECHNICAL FIELD

The present disclosure relates to a field device, a measurement method, and a non-transitory computer readable medium.

### BACKGROUND

Technology for performing predictive diagnosis of a field device having detectors that detect physical quantities and output detection signals is known. For example, Patent Literature (PTL) 1 discloses a predictive diagnosis method that uses a vortex flowmeter and a device management tool that is communicably connected to the output end of the vortex flowmeter. The vortex flowmeter uses a first vortex signal and a second vortex signal, outputted from two detectors that detect vortex signals generated by a vortex generator, to output a vortex flow signal together with a signal ratio between the first vortex signal and the second vortex signal. With the predictive diagnosis method disclosed in PTL 1, the signal ratio is collected by the device management tool to perform predictive diagnosis of blockage of the vortex flowmeter.
PTL 2 relates to a process control system including one or a plurality of field devices configured to be placed in a plant; and a control apparatus configured to perform at least one of input and output on the field device to control the plant; and a change trend calculation device configured to calculate a change trend in time-series data including an observed value at each point in time of the field device.
PTL 3 relates to rotating and compressing Log Files in Linux with Logrotate. PTL 4 relates to a time-series data diagnosing/compressing method.
PTL 5 relates to methods and systems for analyzing the degradation and failure of mechanical systems.
PTL 6 relates to data collection system and method, and method for reducing the quantity of measurement data.

### CITATION LIST

### Patent Literature

PTL 1: JP 2008-070292 A
PTL 2: US 2018/314243 A1
PTL 3: Kiarie James: "How to Rotate and Compress Log Files in Linux with Logrotate", 26 May 2020, pages 1-20, URL: https://www.linuxtechi.com/manage-linux-log-files-using-logrotate/
PTL 4: US 2013/0971 28 A1
PTL 5: US 2004/030524 A1
PTL 6: EP 3 309 682 A1

### SUMMARY

The present invention is defined by the independent claims. Preferred embodiments are defined by the dependent claims. Further aspects are provided for facilitating the understanding of the invention. For long-term confirmation of a measured trend, indicating the temporal change in parameters used for predictive diagnosis of a field device such as a vortex flowmeter, with a predictive diagnosis method such as the one described in PTL 1, it is necessary to communicably connect the field device continuously, for an extended period of time, with an external device such as a device management tool. Parameters need to be collected from the field device while the field device and the external device are communicably connected to each other. For example, if the communicable connection between the field device and the external device cannot be maintained, the user needs to store a massive amount of past data. This has reduced user convenience when predictive diagnosis of a field device is performed.

It would be helpful to provide a field device, a measurement method, and a non-transitory computer readable medium that improve user convenience when predictive diagnosis of a field device is performed.

A field device according to an embodiment includes a detector configured to detect a physical quantity and output a detection signal; a controller configured to calculate, based on the detection signal, data for a measured trend indicating a temporal change in a parameter used for predictive diagnosis of the field device; and a memory configured to store the data, wherein the controller stores the data in the memory at time intervals from a start time of measurement in the field device to a current time and executes a compression process on the data stored in the memory upon an amount of the data stored in the memory reaching an upper limit of a storage capacity of the memory.

This configuration increases user convenience when predictive diagnosis of a field device is performed. For example, by the field device itself including a memory that stores the data for the measured trend indicating the temporal change in the parameters used for the predictive diagnosis of the field device, parameters can be stored by the field device alone as a measured trend over an extended period of time. This eliminates the need for the field device to be communicably connected continuously to an external device over an extended period of time for long-term confirmation of a measured trend. Even if the communicable connection between the field device and the external device cannot be maintained, the user does not need to store a massive amount of past data.

According to the invention, the controller calculates a predicted trend indicating a temporal change in the parameter from the current time onward based on a prediction algorithm that uses the measured trend. This enables the field device to predict future trends based on the data for long-term measured trends stored in the storage inside the field device. Predictive diagnosis based on future trends thus becomes possible through operation of the field device alone. Unlike with known technology, the field device can achieve predictive diagnosis without being combined with an external device.

In an embodiment, the controller may calculate the predicted trend based on at least one prediction algorithm selected by a user from among a plurality of prediction algorithms. This enables the field device to provide the user with the optimal predicted trend for the user for performing predictive diagnosis based on the prediction algorithm desired by the user. The user can select the optimal prediction algorithm according to the characteristics of the temporal change in the parameter. This configuration further increases user convenience when predictive diagnosis of a field device is performed.

In an embodiment, the controller may calculate an initial change point in the measured trend and calculate the predicted trend based on the prediction algorithm that takes a trend identical to the measured trend from a time corresponding to the change point to the current time as the predicted trend.

By calculating the predicted trend based on such a prediction algorithm, the field device can predict the trend with a high degree of accuracy in the case in which, for example, the parameter decays periodically. For example, if the flow rate is low during the first half of the week and high during the second half, then the week overall exhibits a constant pattern, with the parameter remaining roughly constant in the first half and decreasing in the second half. The field device can calculate the predicted trend for one week out from the present, even in such a case in which the parameter decays periodically.

According to the invention, the field device includes an output interface configured to output information to a user, and the controller may display the measured trend and the predicted trend as a graph on the output interface and/or an external device communicably connected to the field device. This enables the user to confirm the measured trend and the predicted trend easily by the output interface and/or the external device, such as the communication device and the display.

In an embodiment, the controller may acquire a determination level and a determination time set by a user, calculate a time, in the predicted trend, corresponding to the determination level as an arrival time, and execute a notification process to notify the user of a result of predictive diagnosis upon determining that a time from the current time until the arrival time is equal to or less than the determination time.

This configuration enables the user easily to recognize a high probability that a sensor will fail or deteriorate beyond a certain level within the determination time set by the user. Such notification by the communication device and display device also enables the user to perform maintenance work, such as cleaning or replacement of the sensor, promptly before the sensor fails or deteriorates beyond a certain level.

In an embodiment, the field device may further include an output interface configured to output information to a user, and the controller may display the calculated arrival time and notification information based on the notification process on the output interface and/or an external device communicably connected to the field device.

By the field device displaying the arrival time on the output interface and/or the external device, the user can learn the arrival time in advance. The user can, in advance, recognize the possible future occurrence of failure of a sensor in the detector or deterioration beyond a certain level. Under normal circumstances, it is difficult for the user to foresee such failure of a sensor or deterioration beyond a certain level. When a lower limit is set for the predicted trend based on the determination level and the arrival time is calculated, the user can easily foresee failure of a sensor or deterioration beyond a certain level. Furthermore, by the field device displaying the notification information, which is based on the notification process, on the output interface and/or the external device, the user can easily recognize visual information indicating a high probability that a sensor will fail or deteriorate beyond a certain level within the determination time set by the user.

In an embodiment, the controller may store the data in the memory in cycles and execute the compression process by averaging first data in one cycle with second data in one or more other cycles. The field device can thereby free up space in the storage that constitutes the memory of the field device and store the data for the measured trend in the memory for an extended period of time. This eliminates the need for the field device and the external device to be communicably connected continuously for an extended period of time.

A measurement method according to an embodiment is a measurement method using a field device and includes detecting a physical quantity and outputting a detection signal; calculating, based on the detection signal, data for a measured trend indicating a temporal change in a parameter used for predictive diagnosis of the field device; storing the data in a memory of the field device at time intervals from a start time of measurement in the field device to a current time; and executing a compression process on the data stored in the memory upon an amount of the data stored in the memory reaching an upper limit of a storage capacity of the memory.

This configuration increases user convenience when predictive diagnosis of a field device is performed. For example, by the field device itself including a memory that stores the data for the measured trend indicating the temporal change in the parameters used for the predictive diagnosis of the field device, parameters can be stored by the field device alone as a measured trend over an extended period of time. This eliminates the need for the field device to be communicably connected continuously to an external device over an extended period of time for long-term confirmation of a measured trend. Even if the communicable connection between the field device and the external device cannot be maintained, the user does not need to store a massive amount of past data.

A non-transitory computer readable medium according to an embodiment stores a program executable by one or more processors to cause a field device to execute functions including detecting a physical quantity and outputting a detection signal; calculating, based on the detection signal, data for a measured trend indicating a temporal change in a parameter used for predictive diagnosis of the field device; storing the data in a memory of the field device at time intervals from a start time of measurement in the field device to a current time; and executing a compression process on the data stored in the memory upon an amount of the data stored in the memory reaching an upper limit of a storage capacity of the memory.

This configuration increases user convenience when predictive diagnosis of a field device is performed. For example, by the field device itself including a memory that stores the data for the measured trend indicating the temporal change in the parameters used for the predictive diagnosis of the field device, parameters can be stored by the field device alone as a measured trend over an extended period of time. This eliminates the need for the field device to be communicably connected continuously to an external device over an extended period of time for long-term confirmation of a measured trend. Even if the communicable connection between the field device and the external device cannot be maintained, the user does not need to store a massive amount of past data.

According to the present disclosure, a field device, a measurement method, and a non-transitory computer readable medium that improve user convenience when predictive diagnosis of a field device is performed can be provided.

### BRIEF DESCRIPTION OF THE DRAWINGS

In the accompanying drawings:
FIG. 1 is a block diagram illustrating an example configuration of a measurement system according to an embodiment;
FIG. 2 is a functional block diagram corresponding to the functions implemented by the controller in FIG. 1;
FIG. 3 is a functional block diagram corresponding to the functions implemented by the memory in FIG. 1;
FIG. 4 is a flowchart illustrating a first example of operations of the field device in FIG. 1;
FIG. 5 is a flowchart illustrating a second example of operations of the field device in FIG. 1;
FIG. 6 is a conceptual diagram illustrating an example of processing by the controller of the field device in FIG. 1;
FIG. 7 illustrates a first example of a graph displayed by the communication device and/or the display device in FIG. 1;
FIG. 8 is a diagram illustrating an example of a prediction algorithm for the predicted trend in FIG. 7; and
FIG. 9 illustrates a second example of a graph displayed by the communication device and/or the display device in FIG. 1.

### DETAILED DESCRIPTION

The problems with known technology are described in greater detail.

For example, a known field device such as the vortex flowmeter described in PTL 1 includes a detector and a converter connected to the detector. The converter includes a signal acquisition interface, a calculator, a control information memory, a communication controller, and a display controller. The converter of the field device is communicably connected to external devices such as a communication device and a display device.

The detector detects a physical quantity and outputs a detection signal to the converter. The signal acquisition interface of the converter acquires the detection signal outputted from the detector. Based on the detection signals acquired by the signal acquisition interface, the calculator calculates measurement information, such as data related to physical quantities, and diagnostic information for use in predictive diagnosis of failure or the like of the detector. The control information memory stores control information for each function of the converter as well as for the detector, the communication device, and the display device. The communication controller controls the transmission and reception of communication data between the converter of the field device and the communication device. The display controller controls the transmission and reception of display data between the converter of the field device and the display device.

The communication device transmits and receives information, such as the above-described measurement information and diagnostic information, along with setting information and the like, to and from the converter of the field device. The display device transmits and receives information, such as the above-described measurement information and diagnostic information, along with setting information and the like, to and from the converter of the field device.

For example, based on the detection signals outputted from the detector, the calculator of the converter calculates data for a measured trend indicating the temporal change in parameters used for predictive diagnosis of the field device. The communication controller of the converter transmits the data calculated by the calculator to the communication device. The display controller of the converter transmits the data calculated by the calculator to the display device. At this time, the converter needs to be communicably connected to the communication device and the display device for an extended period of time in order to transmit the data for the measured trend. The communication device and the display device display the data received from the converter of the field device according to their respective user interfaces. The communication device and the display device need to be communicably connected to the converter of the field device for an extended period of time in order to display the data for the measured trend.

For long-term confirmation of a measured trend, indicating the temporal change in parameters used for predictive diagnosis of a field device such as a vortex flowmeter, with the above-described known technology, it is necessary to communicably connect the field device continuously, for an extended period of time, with an external device. Parameters need to be collected from the field device while the field device and the external device are communicably connected to each other. For example, if the communicable connection between the field device and the external device cannot be maintained, the user needs to store a massive amount of past data, which reduces user convenience when predictive diagnosis of a field device is performed.

Additionally, in order to predict future temporal change in parameters used for predictive diagnosis of field devices, it is necessary to combine an external device, such as a communication device, with the field device. The communication device uses the long-term measured trend of the parameters collected from the field device to predict a future trend based on a predetermined prediction algorithm. With known technology, predictive diagnosis can be achieved by combining a field device with an external device, but it is difficult to achieve such predictive diagnosis with field devices alone.

A field device 10, a measurement method, and a non-transitory computer readable medium capable of resolving these problems are described below. Embodiments of the present disclosure are described with reference to the drawings.

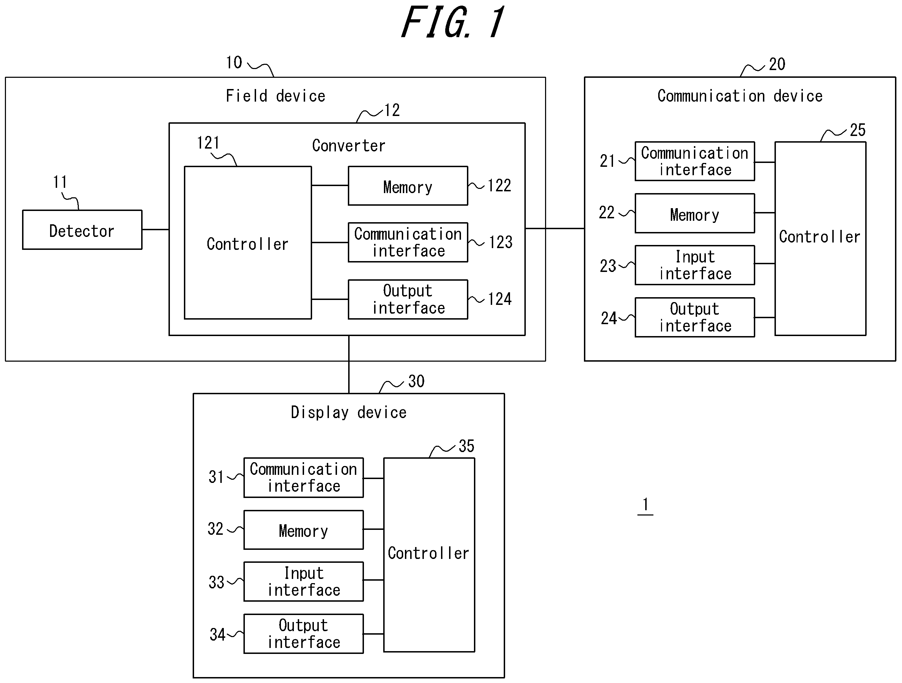

FIG. 1 is a block diagram illustrating an example configuration of a measurement system 1 according to an embodiment. The configuration of the measurement system 1 according to an embodiment is mainly described with reference to FIG. 1. An output interface 124 is illustrated in FIG. 1 but will be explained at the end of this description as a variation. Here, a converter 12 is described as including only a controller 121, a memory 122, and a communication interface 123.

The measurement system 1 includes a field device 10, a communication device 20, and a display device 30. The field device 10 and the communication device 20 are communicably connected to each other. The field device 10 and the display device 30 are communicably connected to each other. The communication protocol between the field device 10 and the communication device 20 and the communication protocol between the field device 10 and the display device 30 may be the same or different.

The field device 10 includes any field device that performs a measurement process on a physical quantity to be measured and acquires a measurement value. In the present description, a "physical quantity" includes, for example, the temperature, pressure, flow rate, and PH of fluids, including gases and liquids, generated at the plant facility where the field device 10 is installed, along with the degree of corrosion, vibration, and the like of the plant facility. These examples are not limiting, and the physical quantity may include state parameters, including temperature, pressure, or the like, associated with actuators such as valves, motors, and relays.

In addition to an industrial plant such as a chemical plant, examples of the "plant facility" in the present description include a plant for managing a well site, such as a gas field or oil field, and the surrounding area. Additional examples of the plant facility may include a plant for managing power generation such as hydropower, thermal power, or nuclear power; a plant for managing environmental power generation such as solar power or wind power; and a plant for managing water and sewage, a dam, or the like.

The field device 10 includes a detector 11 and the converter 12 connected to the detector 11. The converter 12 includes the controller 121, the memory 122, and the communication interface 123. The converter 12 is communicably connected to external devices such as the communication device 20 and the display device 30.

The detector 11 includes any sensor that detects a physical quantity and outputs a detection signal to the converter 12. The detector 11 includes, for example, a temperature sensor, a pressure sensor, a flow sensor, a PH sensor, a corrosivity sensor, and a vibration quantity sensor.

The controller 121 of the converter 12 includes one or more processors. More specifically, the controller 121 includes a general purpose processor or a processor dedicated to a specific process. The controller 121 is connected to each component configuring the field device 10 and controls and manages the field device 10 overall, starting with the components thereof.

Based on the detection signal outputted from the detector 11, the controller 121 calculates data for a measured trend indicating the temporal change in a parameter used for predictive diagnosis of the field device 10. In the present description, "predictive diagnosis" includes, for example, diagnosing failure of a sensor, or deterioration beyond a certain level, that is likely to occur in the future based on a predetermined factor. The "predetermined factor" includes, for example, deterioration over time of the sensor configuring the detector 11, as well as factors such as wear and adhesion of foreign matter through contact between fluids and the sensor. The "parameter" is an index for predictive diagnosis and includes, for example, the sensitivity of the sensor configuring the detector 11, the signal strength of the detection signal outputted from the detector 11, and the above-described physical quantity itself.

The memory 122 includes any appropriate storage module, such as a hard disk drive (HDD), a solid state drive (SSD), an electrically erasable programmable read-only memory (EEPROM), a read-only memory (ROM), and a random access memory (RAM). The memory 122 stores information necessary to realize the operations of the field device 10. For example, the memory 122 stores the above-described data for the measured trend. For example, the memory 122 stores firmware necessary to realize the operations of the field device 10. The memory 122 may function as a main storage apparatus, an auxiliary storage apparatus, or a cache memory. The memory 122 is not limited to being internal to the field device 10 and may include an external storage module connected through a digital input/output port or the like, such as universal serial bus (USB).

The communication interface 123 includes any communication interface compliant with an appropriate wired or wireless communication protocol. More specifically, the communication interface 123 includes a communication interface compliant with a communication protocol used for communication with the communication device 20 and with the display device 30. For example, the communication interface 123 includes a first communication circuit used for communication with the communication device 20 and a second communication circuit used for communication with the display device 30.

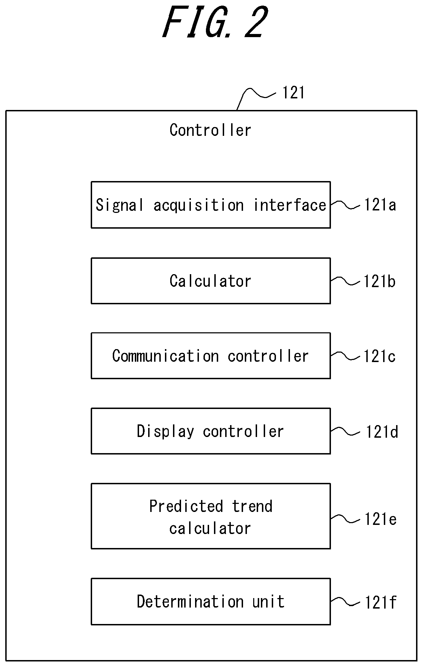

FIG. 2 is a functional block diagram corresponding to the functions implemented by the controller 121 in FIG. 1. With reference to FIG. 2, each functional component included in the controller 121 is explained in more detail. The controller 121 includes a signal acquisition interface 121a, a calculator 121b, a communication controller 121c, and a display controller 121d. In addition to these functional components, the controller 121 further includes a predicted trend calculator 121e and a determination unit 121f.

The signal acquisition interface 121a acquires the detection signal outputted from the detector 11. Based on the detection signals acquired by the signal acquisition interface 121a, the calculator 121b calculates measurement information, such as data related to physical quantities, and diagnostic information for use in predictive diagnosis of failure or the like of the detector 11. For example, based on the detection signal acquired by the signal acquisition interface 121a, the calculator 121b calculates data for a measured trend indicating the temporal change in a parameter used for predictive diagnosis of the field device 10. In the present description, the "diagnostic information" includes, for example, a measured trend indicating the temporal change in a parameter used for predictive diagnosis of the field device 10.

The communication controller 121c controls the transmission and reception of communication data between the converter 12 of the field device 10 and the communication device 20. The display controller 121d controls the transmission and reception of display data between the converter 12 of the field device 10 and the display device 30.

The predicted trend calculator 121e calculates a predicted trend indicating a temporal change in the parameter from the current time onward based on a prediction algorithm that uses the measured trend. The determination unit 121f determines whether it is necessary to notify the user of the result of the predictive diagnosis based on the predicted trend and on a determination level and determination time set by the user. In the present description, the "current time" includes, for example, the date and time. The "determination level" includes, for example, the level of a parameter to be determined as failure of the sensor or deterioration beyond a certain level, and is empirically determined by the user in advance. The "determination time" includes, for example, the time that the user wishes to have as an advance warning before failure, or deterioration beyond a certain level, of the sensor. For example, the determination time includes the minimum time that the field device 10 should be kept in operation from the current time. As an example, the determination time includes the time from preparation through performance of maintenance on the field device 10.

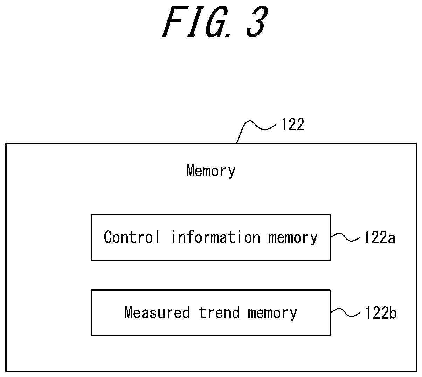

FIG. 3 is a functional block diagram corresponding to the functions implemented by the memory 122 in FIG. 1. With reference to FIG. 3, each functional component included in the memory 122 is explained in more detail. The memory 122 includes a control information memory 122a and a measured trend memory 122b.

The control information memory 122a stores control information for each function of the converter 12 as well as for the detector 11, the communication device 20, and the display device 30. The measured trend memory 122b stores the data, from each elapsed time interval, for the measured trend calculated by the calculator 121b.

Referring again to FIG. 1, the configuration of the communication device 20 is mainly described.

The communication device 20 includes any appropriate information processing device that transmits and receives information, such as the above-described measurement information and diagnostic information, along with setting information and the like, to and from the converter 12 of the field device 10. The communication device 20 may be any general purpose electronic device such as a smartphone, cell phone, tablet personal computer (PC), desktop computer, or mobile computer, or may be a dedicated information processing device specialized for the configuration of the measurement system 1.

The communication device 20 includes a communication interface 21, a memory 22, an input interface 23, an output interface 24, and a controller 25.

The communication interface 21 includes any communication interface compliant with a suitable wired or wireless communication protocol. More specifically, the communication interface 21 includes a communication interface compliant with a communication protocol used for communication with the field device 10.

The memory 22 includes any appropriate storage module, such as an HDD, SSD, EEPROM, ROM, or RAM. The memory 22 stores information necessary to realize the operations of the communication device 20. The memory 22 may function as a main storage apparatus, an auxiliary storage apparatus, or a cache memory. The memory 22 is not limited to being internal to the communication device 20 and may include an external storage module connected through a digital input/output port or the like, such as USB.

The input interface 23 includes any appropriate input interface that receives an input operation by the user of the communication device 20 and acquires input information based on the user operation. The input interface 23 may, for example, include physical keys, capacitive keys, a touchscreen provided integrally with an LCD monitor, or a microphone that accepts audio input. The input interface 23 outputs the acquired input information to the controller 25.

The output interface 24 includes any appropriate output interface that outputs information to the user of the communication device 20. The output interface 24 may, for example, include any appropriate output interface that affects the user's vision and/or hearing. The output interface 24 may, for example, include any appropriate image output interface that primarily affects the user's vision. For example, the output interface 24 may include an LCD monitor. The output interface 24 may, for example, include any appropriate audio output interface that primarily affects the user's hearing.

The controller 25 includes one or more processors. More specifically, the controller 25 includes a general purpose processor or a processor dedicated to a specific process. The controller 25 is connected to each component configuring the communication device 20 and controls and manages the communication device 20 overall, starting with the components thereof.

Referring to FIG. 1, the configuration of the display device 30 is now mainly described.

The display device 30 includes any appropriate information processing device that transmits and receives information, such as the above-described measurement information and diagnostic information, along with setting information and the like, to and from the converter 12 of the field device 10. The display device 30 may be any general purpose electronic device equipped with a monitor or may be a dedicated information processing device specialized for the configuration of the measurement system 1.

The display device 30 includes a communication interface 31, a memory 32, an input interface 33, an output interface 34, and a controller 35.

The communication interface 31 includes any communication interface compliant with a suitable wired or wireless communication protocol. More specifically, the communication interface 31 includes a communication interface compliant with a communication protocol used for communication with the field device 10.

The memory 32 includes any appropriate storage module, such as an HDD, SSD, EEPROM, ROM, or RAM. The memory 32 stores information necessary to realize the operations of the display device 30. The memory 32 may function as a main storage apparatus, an auxiliary storage apparatus, or a cache memory. The memory 32 is not limited to being internal to the display device 30 and may include an external storage module connected through a digital input/output port or the like, such as USB.

The input interface 33 includes any appropriate input interface that receives an input operation by the user of the display device 30 and acquires input information based on the user operation. The input interface 33 may, for example, include physical keys, capacitive keys, a touchscreen provided integrally with an LCD monitor, or a microphone that accepts audio input. The input interface 33 outputs the acquired input information to the controller 35.

The output interface 34 includes any appropriate output interface that outputs information to the user of the display device 30. The output interface 34 includes, for example, any appropriate image output interface that primarily affects the user's vision. For example, the output interface 34 may include an LCD monitor. This example is not limiting, however, and the output interface 34 may, for example, include any appropriate audio output interface that also affects the user's hearing in addition to the user's vision.

The controller 35 includes one or more processors. More specifically, the controller 35 includes a general purpose processor or a processor dedicated to a specific process. The controller 35 is connected to each component configuring the display device 30 and controls and manages the display device 30 overall, starting with the components thereof.

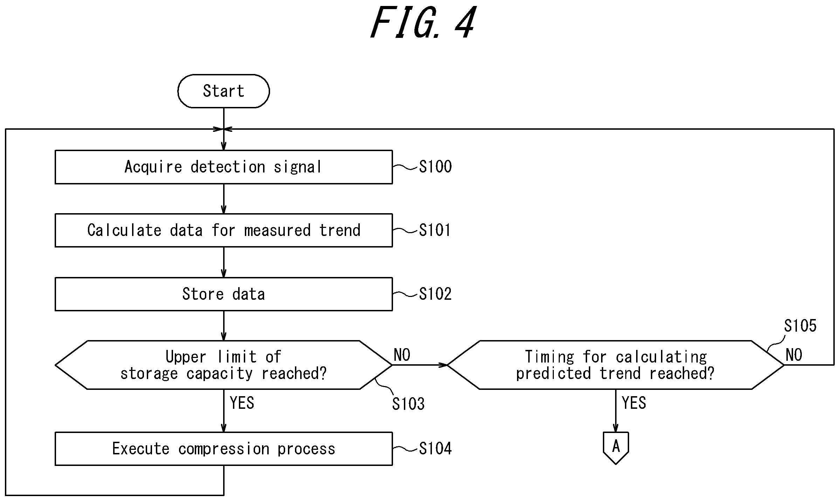

FIG. 4 is a flowchart illustrating a first example of operations of the field device 10 in FIG. 1. Referring to FIG. 4, an example of a measurement method performed by the field device 10 is now mainly described.

In step S100, the signal acquisition interface 121a of the controller 121 acquires a detection signal outputted from the detector 11.

In step S101, based on the detection signal acquired in step S100, the calculator 121b of the controller 121 calculates data for a measured trend indicting the temporal change in a parameter used for predictive diagnosis of the field device 10.

In step S102, the calculator 121b of the controller 121 stores the data calculated in step S101 in the memory 122 at time intervals from the start time of measurement in the field device 10 to the current time.

In step S103, the calculator 121b of the controller 121 determines whether the amount of the data stored in the memory 122 has reached the upper limit of the storage capacity of the memory 122. Upon determining that the upper limit of the storage capacity has been reached, the calculator 121b executes the process of step S104. Upon determining that the upper limit of the storage capacity has not been reached, the calculator 121b executes the process of step S105. In the present description, the "upper limit of the storage capacity" may, for example, include all of the storage areas available for the memory 122, storage areas that are less than all of the available storage areas by a unit of the data storage capacity in the memory 122, or the like.

In step S104, after determining in step S103 that the amount of the data stored in the memory 122 has reached the upper limit of the storage capacity of the memory 122, the calculator 121b of the controller 121 executes a compression process on the data stored in the memory 122.

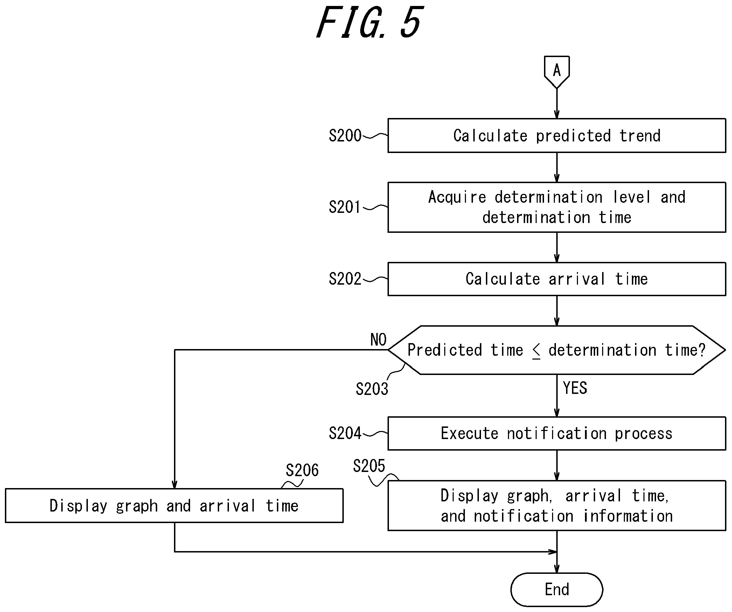

In step S105, after determining in step S103 that the amount of the data stored in the memory 122 has not reached the upper limit of the storage capacity of the memory 122, the calculator 121b of the controller 121 determines whether the timing for calculating the predicted trend has been reached. For example, the calculator 121b may perform this determination process based on the measurement time that the user sets in advance using the input interface 23 of the communication device 20 or the input interface 33 of the display device 30. Such a measurement time corresponds to the time from the start time to the current time. When the calculator 121b determines that the timing for calculating the predicted trend has been reached, the controller 121 executes the process of step S200 in FIG. 5, described below. In other words, the controller 121 executes the information processing method for predictive diagnosis, described below with reference to FIG. 5, at the current time. When the calculator 121b determines that the timing for calculating the predicted trend has not been reached, the controller 121 again executes the process of step S100.

FIG. 5 is a flowchart illustrating a second example of operations of the field device 10 in FIG. 1. Referring to FIG. 5, an example of an information processing method for predictive diagnosis performed by the field device 10 is now mainly described.

In step S200, the predicted trend calculator 121e of the controller 121 calculates a predicted trend indicating a temporal change in a parameter from the current time onward based on a prediction algorithm, using the measured trend based on the data stored in the memory 122 in step S102 of FIG. 4. At this time, the predicted trend calculator 121e calculates the predicted trend based on the prediction algorithm while referring to the value of any parameter for any time while the field device 10 is in the operating state, that is, using the measured trend based on any data stored in the memory 122 for any time.

In step S201, the determination unit 121f of the controller 121 acquires the determination level and determination time set by the user. For example, the determination unit 121f acquires, via the communication controller 121c, the determination level and determination time inputted by the user using the input interface 23 of the communication device 20. For example, the determination unit 121f acquires, via the display controller 121d, the determination level and determination time inputted by the user using the input interface 33 of the display device 30.

In step S202, the calculator 121b of the controller 121 calculates the time corresponding to the determination level, acquired in step S201, indicating the value of the parameter in the predicted trend calculated in step S200 as the arrival time. In the present description, the "arrival time" includes, for example, the date and time.

In step S203, the determination unit 121f of the controller 121 determines whether a prediction time corresponding to the time from the current time to the arrival time is equal to or less than the determination time acquired in step S201. When determining that the prediction time is equal to or less than the determination time, the determination unit 121f executes the process of step S204. When determining that the prediction time is longer than the determination time, the determination unit 121f executes the process of step S206.

When it is determined in step S203 that the prediction time is equal to or less than the determination time, the determination unit 121f of the controller 121 executes a notification process in step S204 for notifying the user of the result of the predictive diagnosis. For example, the determination unit 121f generates notification information for notifying the user of the result of the predictive diagnosis via the output interface 24 of the communication device 20. For example, the determination unit 121f generates notification information for notifying the user of the result of the predictive diagnosis via the output interface 34 of the display device 30. The result of the predictive diagnosis includes a result indicating a high probability that a sensor will fail or deteriorate beyond a certain level, based on a predetermined factor, within the determination time set by the user.

In step S205, the controller 121 displays the measured trend based on the data stored in the memory 122 in step S102 of FIG. 4 and the predicted trend calculated in step S200 as a graph on an external device. For example, the communication controller 121c of the controller 121 transmits information on the measured trend and the predicted trend to the communication device 20. For example, the display controller 121d of the controller 121 transmits information on the measured trend and the predicted trend to the display device 30.

Additionally, the controller 121 displays the arrival time calculated in step S202 on an external device. For example, the communication controller 121c of the controller 121 transmits information on the arrival time to the communication device 20. For example, the display controller 121d of the controller 121 transmits information on the arrival time to the display device 30.

Additionally, the controller 121 displays the notification information, which is based on the notification process, in step S204 on an external device. For example, the communication controller 121c of the controller 121 transmits the notification information generated in step S204 to the communication device 20. For example, the display controller 121d of the controller 121 transmits the notification information generated in step S204 to the display device 30.

As a result, the user can confirm the measured trend and predicted trend, the arrival time, and the result of predictive diagnosis based on the output interface 24 of the communication device 20 and/or the output interface 34 of the display device 30.

In step S206, when it is determined in step S203 that the prediction time is longer than the determination time, the controller 121 displays the measured trend based on the data stored in the memory 122 in step S102 of FIG. 4 and the predicted trend calculated in step S200 as a graph on an external device. For example, the communication controller 121c of the controller 121 transmits information on the measured trend and the predicted trend to the communication device 20. For example, the display controller 121d of the controller 121 transmits information on the measured trend and the predicted trend to the display device 30.

Additionally, the controller 121 displays the arrival time calculated in step S202 on an external device. For example, the communication controller 121c of the controller 121 transmits information on the arrival time to the communication device 20. For example, the display controller 121d of the controller 121 transmits information on the arrival time to the display device 30.

As a result, the user can confirm the measured trend and predicted trend, along with the arrival time, based on the output interface 24 of the communication device 20 and/or the output interface 34 of the display device 30.

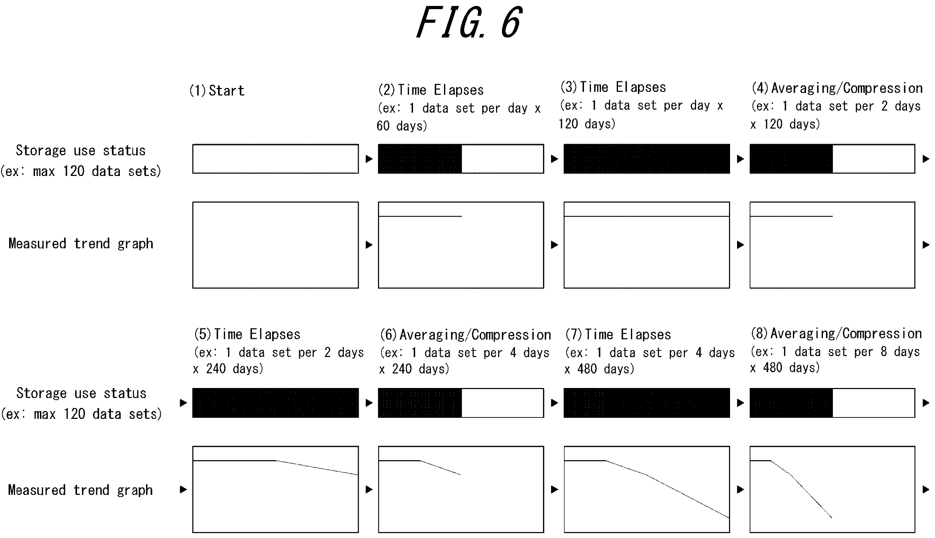

FIG. 6 is a conceptual diagram illustrating an example of processing by the controller 121 of the field device 10 in FIG. 1. With reference to FIG. 6, the data compression process executed by the calculator 121b of the controller 121 in step S104 of FIG. 4 is described in more detail.

The calculator 121b of the controller 121 stores the data for the measured trend, calculated in step S101 of FIG. 4, in the memory 122 at time intervals from the start time of measurement in the field device 10 to the current time. For example, the calculator 121b stores the data for the measured trend in the memory in cycles. When determining that the amount of the data stored in the memory 122 has reached the upper limit of the storage capacity of the memory 122, the calculator 121b executes the compression process illustrated in step S104 of FIG. 4 by averaging first data in one cycle with second data in one or more other cycles.

For example, suppose that the maximum capacity of the storage constituting the memory 122 is 120 data sets. At the time of (1) Start in FIG. 6, no data whatsoever is stored in the storage constituting the memory 122. As illustrated by (2) Time Elapses, the calculator 121b stores the data for the measured trend in the memory 122 for each one-day cycle immediately after the start time has passed. The calculator 121b continues to store the values of parameters acquired at any unit times over a day as one data set in the memory 122 for 120 days.

When the amount of the data stored in the memory 122 reaches the upper limit of the storage capacity of the memory 122 on the 120^{th} day, as illustrated by (3) Time Elapses, the calculator 121b executes a compression process by averaging first data on a predetermined day with second data on another day to yield one data set, as illustrated by (4) Averaging/Compression. For example, the first data and the second data may be data in consecutive cycles or may be data in cycles that are not consecutive. The target to be averaged with the first data on a predetermined day as one data set is not limited to the second data on another day. The target to be averaged may include second data in a plurality of other cycles, i.e., second data on two or more other days.

In this way, the calculator 121b frees storage space. Furthermore, the calculator 121b changes the storage cycle in order to achieve storage of long-term measured trend data within the storage capacity constraints. For example, the calculator 121b increases the subsequent storage cycle from one day to two days.

The calculator 121b stores the data for the measured trend in the memory 122 for each two-day cycle immediately after the 120^{th} day has passed. The calculator 121b continues to store the values of parameters acquired at any unit times over two days as one data set in the memory 122 for another 120 days.

When the amount of the data stored in the memory 122 reaches the upper limit of the storage capacity of the memory 122 on the 240^{th} day, as illustrated by (5) Time Elapses, the calculator 121b executes a compression process by averaging first data on two predetermined days with second data on two other days to yield one data set, as illustrated by (6) Averaging/Compression. In this way, the calculator 121b frees storage space. Furthermore, the calculator 121b changes the storage cycle in order to achieve storage of long-term measured trend data within the storage capacity constraints. For example, the calculator 121b increases the subsequent storage cycle from two days to four days.

The calculator 121b stores the data for the measured trend in the memory 122 for each four-day cycle immediately after the 240^{th} day has passed. The calculator 121b continues to store the values of parameters acquired at any unit times over four days as one data set in the memory 122 for another 240 days.

When the amount of the data stored in the memory 122 reaches the upper limit of the storage capacity of the memory 122 on the 480^{th} day, as illustrated by (7) Time Elapses, the calculator 121b executes a compression process by averaging first data on four predetermined days with second data on four other days to yield one data set, as illustrated by (8) Averaging/Compression. In this way, the calculator 121b frees storage space. Furthermore, the calculator 121b changes the storage cycle in order to achieve storage of long-term measured trend data within the storage capacity constraints. For example, the calculator 121b increases the subsequent storage cycle from four days to eight days.

The calculator 121b repeats the above process flow from when 480 days have passed until the current time.

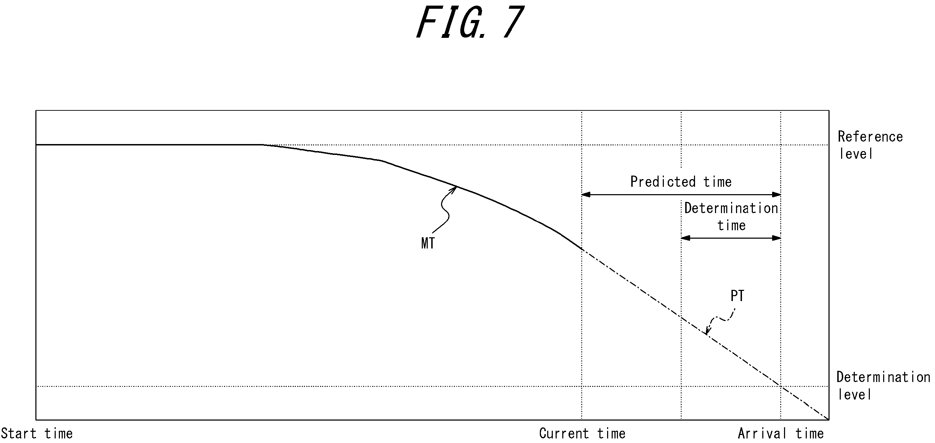

FIG. 7 illustrates a first example of a graph displayed by the communication device 20 and/or the display device 30 in FIG. 1. With reference to FIG. 7, the first example of a graph, displayed by the output interface 24 of the communication device 20 and/or the output interface 34 of the display device 30, illustrating the measured trend and predicted trend along with the arrival time will mainly be explained. The graph displayed by the output interface 24 of the communication device 20 is mainly explained, but the same explanation applies to the output interface 34 of the display device 30.

For example, the controller 25 of the communication device 20 displays the graph illustrated in FIG. 7 on the output interface 24 based on the information on the measured trend and predicted trend, along with information on the arrival time, received from the converter 12 via the communication interface 21. At this time, the controller 25 may also display the determination level and determination time, inputted by the user using the input interface 23, on the graph.

For example, in FIG. 7, the vertical axis represents the value of a parameter. The horizontal axis represents time. The solid line indicates the measured trend MT. The dashed dotted line extending from the measured trend MT beyond the current time indicates the predicted trend PT. The dotted vertical lines indicate, from left to right, the current time, the determination time, and the arrival time. The prediction time illustrated in FIG. 7 corresponds to the time from the current time to the arrival time. The dotted horizontal lines indicate, in order from top to bottom, the reference level and determination level of the parameter. In the present description, the "reference level" includes the level of a parameter at which it can be determined that no deterioration or failure whatsoever has occurred in a sensor that is, for example, new.

For example, in a case in which the user selects a plurality of prediction algorithms, the output interface 24 of the communication device 20 may display a plurality of predicted trends PT overlaid on the same screen, or a plurality of predicted trends PT separately on different screens. The output interface 24 of the communication device 20 and the output interface 34 of the display device 30 may display the plurality of predicted trends PT separately.

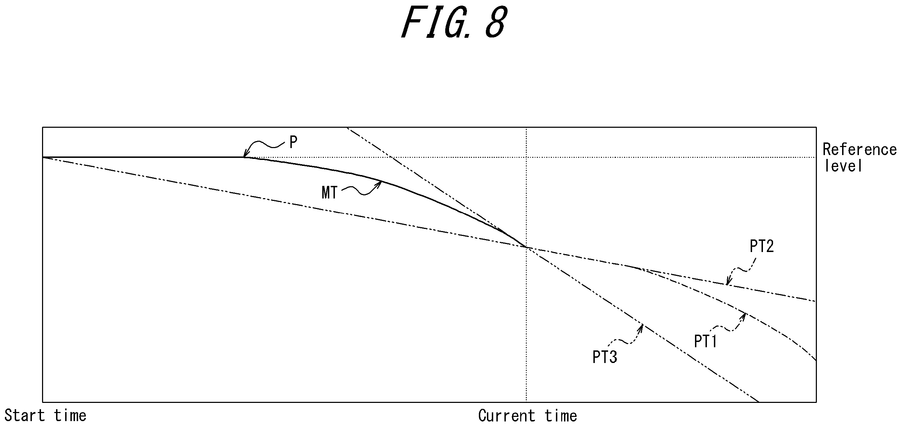

FIG. 8 is a diagram illustrating an example of a prediction algorithm for the predicted trend PT in FIG. 7. With reference to FIG. 8, the prediction algorithm when the predicted trend calculator 121e of the controller 121 calculates the predicted trend PT is mainly explained.

The predicted trend calculator 121e analyzes the measured trend MT up to the current time based on the data stored in the measured trend memory 122b and calculates the predicted trend PT indicating a temporal change in the parameter predicted for the future. For example, the predicted trend calculator 121e may calculate the predicted trend PT based on at least one prediction algorithm selected by the user from among a plurality of prediction algorithms. The selection of the prediction algorithm by the user may, for example, be made using the input interface 23 of the communication device 20 or the input interface 33 of the display device 30.

For example, the predicted trend calculator 121e calculates a predicted trend PT1 based on a first prediction algorithm. For example, the first prediction algorithm includes calculating an initial change point P in the measured trend MT and taking a trend identical to the measured trend MT from the time corresponding to the change point P to the current time as the predicted trend PT1. In other words, the first prediction algorithm includes an iterative approximation from the change point P onward.

For example, the predicted trend calculator 121e calculates the change point P based on the difference from the reference level of the parameter. More specifically, the predicted trend calculator 121e calculates the time at which a certain value difference begins to occur between the value of the parameter and the reference level at the target time in the measured trend MT as the change point P. At this time, the reference level may, for example, be the value of the parameter at the start time, or the average value of the parameter when the parameter fluctuates within a predetermined range between the start time and a predetermined time.

This example is not limiting, and the predicted trend calculator 121e may calculate the time at which a certain percentage difference begins to occur between the value of the parameter and the reference level at the target time in the measured trend MT as the change point P. At this time, the reference level may, for example, be the value of the parameter at the start time, or the difference between the maximum and minimum values of the parameter when the parameter fluctuates within a predetermined range between the start time and a predetermined time.

The above explanation is not limiting, and the predicted trend calculator 121e may calculate the time at which a certain value or a certain percentage difference begins to occur between the value of the parameter at the target time and the value of the parameter at the previous time in the measured trend MT as the change point P.

For example, the predicted trend calculator 121e calculates a predicted trend PT2 based on a second prediction algorithm. For example, the second prediction algorithm includes connecting the start point of the measured trend MT corresponding to the start time and the point of the measured trend MT corresponding to the current time with a straight line and taking the extension of the straight line as the predicted trend PT2. In other words, the second prediction algorithm includes a linear approximation.

For example, the predicted trend calculator 121e calculates a predicted trend PT3 based on a third prediction algorithm. For example, the third prediction algorithm includes taking the amount of change in the measured trend MT at the current time, i.e., a tangent to the point of the measured trend MT corresponding to the current time, as the predicted trend PT3. In other words, the third prediction algorithm includes a linear approximation.

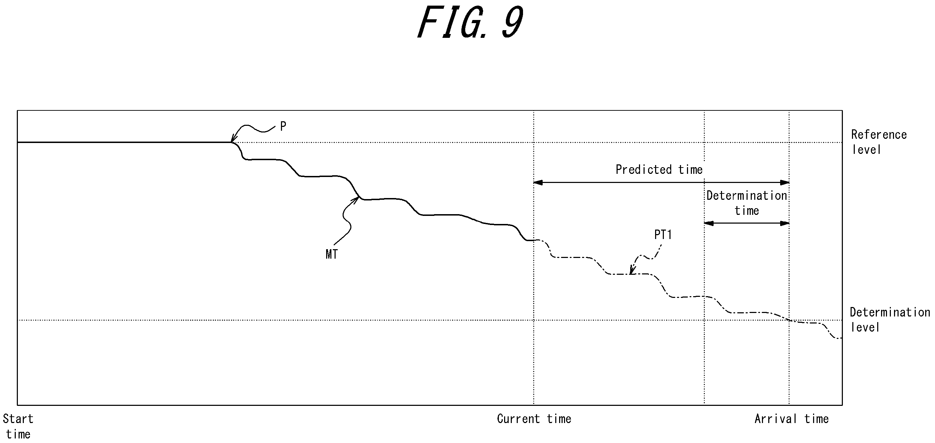

FIG. 9 illustrates a second example of a graph displayed by the communication device 20 and/or the display device 30 in FIG. 1. With reference to FIG. 9, the second example of a graph, displayed on the output interface 24 of the communication device 20 and/or the output interface 34 of the display device 30, illustrating the measured trend and predicted trend along with the arrival time will mainly be explained. The graph displayed by the output interface 24 of the communication device 20 is mainly explained below, but the same explanation applies to the output interface 34 of the display device 30.

For example, the controller 25 of the communication device 20 displays the graph illustrated in FIG. 9 on the output interface 24 based on the information on the measured trend and predicted trend, along with information on the arrival time, received from the converter 12 via the communication interface 21. At this time, the controller 25 may also display the determination level and determination time, inputted by the user using the input interface 23, on the graph.

For example, in FIG. 9, the vertical axis represents the value of a parameter. The horizontal axis represents time. The solid line indicates the measured trend MT. The dashed dotted line extending from the measured trend MT beyond the current time indicates the predicted trend PT1. The dotted vertical lines indicate, from left to right, the current time, the determination time, and the arrival time. The prediction time illustrated in FIG. 9 corresponds to the time from the current time to the arrival time. The dotted horizontal lines indicate, in order from top to bottom, the reference level and determination level of the parameter.

For example, in the case in which the parameter decays periodically as illustrated in FIG. 9, the predicted trend calculator 121e can calculate the predicted trend PT1 with high accuracy based on the first prediction algorithm described using FIG. 8. On the other hand, in the case in which the parameter decays rapidly in a curvilinear manner as illustrated in FIG. 7, the predicted trend calculator 121e can calculate the predicted trend PT3 with high accuracy based on the third prediction algorithm described using FIG. 8. In the case in which the parameter decays linearly, unlike FIG. 7 or FIG. 9, the predicted trend calculator 121e can calculate the predicted trend PT2 with high accuracy based on the second prediction algorithm described using FIG. 8.

As illustrated in FIGS. 7 and 9, the controller 121 executes predictive diagnosis based on the determination level and determination time, inputted by the user using the input interface 23 of the communication device 20 or the input interface 33 of the display device 30, and the predicted trend PT. More specifically, the calculator 121b of the controller 121 calculates the time corresponding to the determination level in the predicted trend PT as the arrival time. The calculator 121b calculates the difference between the arrival time at which the predicted trend PT will exceed the determination level and the current time as the prediction time.

The determination unit 121f of the controller 121 compares the prediction time calculated by the calculator 121b with the determination time. Upon determining that the prediction time is equal to or less than the determination time, the determination unit 121f executes a notification process for notifying the user of the result of the predictive diagnosis. As a result, the output interface 24 of the communication device 20 or the output interface 34 of the display device 30 outputs the result of the predictive diagnosis to the user as visual information, such as an image containing characters or the like, and/or auditory information, such as an alarm. On the other hand, upon determining that the prediction time is longer than the determination time, the determination unit 121f does not execute a notification process for notifying the user of the result of the predictive diagnosis.

According to the above embodiment, user convenience increases when predictive diagnosis of the field device 10 is performed. For example, by the field device 10 itself including the memory 122 that stores the data for the measured trend indicating the temporal change in the parameters used for the predictive diagnosis of the field device 10, parameters can be stored by the field device 10 alone as a measured trend over an extended period of time. This eliminates the need for the field device 10 to be communicably connected continuously to an external device over an extended period of time for long-term confirmation of the measured trend. Even if the communicable connection between the field device 10 and the external device cannot be maintained, the user does not need to store a massive amount of past data.

By calculating the predicted trend based on a prediction algorithm that uses the measured trend, the field device 10 can predict future trends based on data for long-term measured trends stored in the storage inside the field device 10. Predictive diagnosis based on future trends thus becomes possible through operation of the field device 10 alone. Unlike with known technology, the field device 10 can achieve predictive diagnosis without being combined with an external device.

By calculating the predicted trend based on at least one prediction algorithm selected by the user from among a plurality of prediction algorithms, the field device 10 can provide the user with the optimal predicted trend for the user for performing predictive diagnosis based on the prediction algorithm desired by the user. The user can select the optimal prediction algorithm according to the characteristics of the temporal change in the parameter. This configuration further increases user convenience when predictive diagnosis of the field device 10 is performed.

By calculating the predicted trend based on the first prediction algorithm, the field device 10 can predict the trend with a high degree of accuracy in the case in which, for example, the parameter decays periodically as illustrated in FIG. 9. For example, it is easy to obtain the measured trend illustrated in FIG. 9 in a case in which the flow rate of the fluid at the installation location of the detector 11 changes periodically from day to day. For example, in a case in which the sensor forming the detector 11 deteriorates due to factors such as wear and adhesion of foreign matter through contact between fluids and the sensor, the change in the parameter is suppressed, so that the parameter becomes substantially constant, if the flow rate of the fluid is small at the installation location of the detector 11. Conversely, if the flow rate of the fluid is large at the installation location of the detector 11, the parameter is reduced due to the flow of the fluid. As a result of the flow rate of the fluid at the installation location of the detector 11 changing periodically from day to day, the parameter alternates periodically in the measured trend between a substantially constant area and a decreasing area.

By the field device 10 displaying the measured trend and predicted trend as a graph on an external device, the user can easily confirm the measured trend and predicted trend using an external device such as the communication device 20 and the display device 30.

The field device 10 executes a notification process to notify the user of the result of the predictive diagnosis upon determining that the time from the current time to the arrival time is less than the determination time. As a result, the user can easily recognize a high probability that a sensor will fail or deteriorate beyond a certain level within the determination time set by the user. Such notification by the communication device 20 and the display device 30 also enables the user to promptly perform maintenance work, such as cleaning or replacement of the sensor, before the sensor fails or deteriorates beyond a certain level.

By the field device 10 displaying the arrival time on the external device, the user can learn the arrival time in advance. The user can, in advance, recognize the possible future occurrence of failure of a sensor in the detector 11 or deterioration beyond a certain level. Under normal circumstances, it is difficult for the user to foresee such failure of a sensor or deterioration beyond a certain level. When a lower limit is set for the predicted trend based on the determination level and the arrival time is calculated, the user can easily foresee failure of a sensor or deterioration beyond a certain level. Furthermore, by the field device 10 displaying the notification information, which is based on the notification process, on the external device, the user can easily recognize visual information indicating a high probability that a sensor will fail or deteriorate beyond a certain level within the determination time set by the user.

By executing a compression process, the field device 10 can free up space in the storage that constitutes the memory 122 of the field device and store the data for the measured trend in the memory 122 for an extended period of time. This eliminates the need for the field device 10 and the external device to be communicably connected continuously for an extended period of time.

For example, the above-described steps in the operations of the field device 10 and the functions and the like included in each step may be rearranged in any logically consistent way. The order of steps may be changed, steps may be combined, and individual steps may be divided.

For example, the present disclosure may also be embodied as a program containing a description of the processing for achieving the functions of the above-described field device 10 or a storage medium with the program recorded thereon. Such embodiments are also to be understood as falling within the scope of the present disclosure.

In the above embodiment, the field device 10 has been described as calculating the predicted trend based on a prediction algorithm using the measured trend, but this example is not limiting. The field device 10 may calculate the predicted trend based on any prediction algorithm. The field device 10 need not calculate the predicted trend. Even in such a case, users may perform predictive diagnosis themselves by estimating the time corresponding to the aforementioned arrival time from the appearance of the measured trend displayed on the external device.

In the above embodiment, the field device 10 has been described as calculating the predicted trend based on at least one prediction algorithm selected by the user from among a plurality of prediction algorithms, but this example is not limiting. The field device 10 may calculate the predicted trend based on any prediction algorithm determined in advance, without accepting a selection of the prediction algorithm from the user.

In the above embodiment, the field device 10 has been described as calculating the initial change point P in the measured trend in the first prediction algorithm, but this example is not limiting. The field device 10 may calculate the change point P at any time. The field device 10 may calculate a trend identical to the measured trend from any time corresponding to such a change point P to the current time as the predicted trend.

In the above embodiment, the field device 10 has been described as displaying the measured trend and the predicted trend as a graph on the external device, but this example is not limiting. The field device 10 may display the measured trend and the predicted trend on the external device in any other form, such as a table.

In the above embodiment, the field device 10 has been described as displaying the calculated arrival time and the notification information, which is based on the notification process, on the external device, but this example is not limiting. The field device 10 need not display these pieces of information on the external device.

In the above embodiment, the field device 10 has been described as executing the compression process by averaging the first data in one cycle with second data in another cycle, but this example is not limiting. The field device 10 may execute the compression process by any appropriate method. For example, the field device 10 may compress the first data and the second data into one data set using the initial value, middle value, or end value in the first data or the initial value, middle value, or end value in the second data.

In the above embodiment, the measurement system 1 has been described as including the communication device 20 and a display device 30 that is different from the communication device 20, but this example is not limiting. Instead of the communication device 20 and the display device 30, the measurement system 1 may include a single information processing device that has the functions of both the communication device 20 and display device 30 described above. Similarly, instead of the field device 10 and the display device 30 that are different apparatuses from each other, the measurement system 1 may include a single information processing device that has the functions of both the field device 10 and display device 30 described above.

In the above embodiment, only the communication device 20 and the display device 30 have been described as including the output interface 24 and the output interface 34, respectively, with the field device 10 not including such an output interface, but this example is not limiting. For example, the converter 12 of the field device 10 may include an output interface 124 that outputs information to the user, similar to the communication device 20 and the display device 30, as illustrated in FIG. 1.

The output interface 124 includes any appropriate output interface that outputs information to the user of the field device 10. The output interface 124 may, for example, include any appropriate output interface that affects the user's vision and/or hearing. The output interface 124 may, for example, include any appropriate image output interface that primarily affects the user's vision. For example, the output interface 124 may include an LCD monitor. The output interface 124 may, for example, include any appropriate audio output interface that primarily affects the user's hearing.

The display controller 121d of the controller 121 may display the measured trend and the predicted trend as a graph on the output interface 124 and/or an external device communicably connected to the field device 10. Similarly, the display controller 121d of the controller 121 may display the calculated arrival time and the notification information, which is based on the notification process, on the output interface 124 and/or an external device communicably connected to the field device 10.

In the above embodiment, the field device 10 has been described as executing predictive diagnosis by setting a lower limit corresponding to the determination level for a parameter that decays in the measured trend and the predicted trend, but this example is not limiting. The field device 10 may perform predictive diagnosis by setting an upper limit corresponding to the determination level for a parameter that increases in the measured trend and the predicted trend. For example, in a case in which a parameter varies sinusoidally in the measured trend, and the amplitude thereof increases slightly with time, the field device 10 may perform predictive diagnosis by setting both an upper limit and a lower limit corresponding to the determination level for the predicted trend based on the first prediction algorithm.

## Claims

1. A field device (10) comprising:
a detector (11) configured to detect a physical quantity and output a detection signal;
a controller (121) configured to calculate, based on the detection signal, trend data for measuring a measured trend indicating a temporal change in a parameter used for predictive diagnosis of the field device (10); and
a memory (122) configured to store the data, wherein
the controller (121) is configured to store the trend data in the memory (122) at time intervals from a start time of measurement in the field device (10) to a current time, to execute a compression process on the data stored in the memory (122) upon an amount of the trend data stored in the memory (122) reaching an upper limit of a storage capacity of the memory (122), to calculate a measured trend data based on the trend data stored in the memory (122), and to calculate a predicted trend indicating a temporal change in the parameter from the current time onward,
the field device further comprising an output interface configured to output information to a user, wherein the controller is configured to display the measured trend and the predicted trend as a graph on the output interface and/or on an external device communicably connected to the field device.

2. The field device (10) of claim 1, wherein the controller is configured to calculate the predicted trend based on at least one prediction algorithm selected by a user from among a plurality of prediction algorithms.

3. The field device (10) of claim 1 or 2, wherein the controller (121) is configured to calculate an initial change point in the measured trend and calculate the predicted trend based on the prediction algorithm of the at least one prediction algorithm selected by the user that takes a trend identical to the measured trend from a time corresponding to the change point to the current time as the predicted trend.

4. The field device (10) of claim 1, wherein the controller (121) is configured to acquire a determination level and a determination time set by a user, calculate a time, in the predicted trend, corresponding to the determination level as an arrival time, and execute a notification process to notify the user of a result of predictive diagnosis upon determining that a time from the current time until the arrival time is equal to or less than the determination time.

5. The field device (10) of claim 4, wherein
the controller (121) is configured to display the calculated arrival time and notification information based on the notification process on the output interface (24) and/or the external device.

6. The field device (10) of any one of claims 1 to 5, wherein the controller (121) is configured to store the data in the memory (122) in cycles and execute the compression process by averaging first data in one cycle with second data in one or more other cycles.

7. A measurement method using a field device (10), the measurement method comprising:
detecting a physical quantity and outputting a detection signal;
calculating, based on the detection signal, trend data for measuring a measured trend indicating a temporal change in a parameter used for predictive diagnosis of the field device (10);
storing the trend data in a memory (122) of the field device (10) at time intervals from a start time of measurement in the field device (10) to a current time;
executing a compression process on the trained data stored in the memory (122) upon an amount of the trend data stored in the memory (122) reaching an upper limit of a storage capacity of the memory (122); and
calculating a measured trend based on the trend data stored in the memory,
calculating a predicted trend indicating a temporal change in the parameter from the current time onward, and
displaying the measured trend and the predicted trend as a graph on an output interface of the field device and/or on an external device communicably connected to the field device.

8. A non-transitory computer readable medium storing a program executable by one or more processors to cause a field device (10) to execute functions according to claim 7.

## Patentansprüche

1. Feldvorrichtung (10), umfassend:
einen Detektor (11), der so konfiguriert ist, dass er eine physikalische Größe detektiert und ein Detektionssignal ausgibt;
eine Steuereinheit (121), die so konfiguriert ist, dass sie auf der Grundlage des Detektionssignals Trenddaten zur Messung eines gemessenen Trends berechnet, der eine zeitliche Änderung eines Parameters angibt, der für die prädiktive Diagnose der Feldvorrichtung (10) verwendet wird; und
einen Speicher (122), der so konfiguriert ist, dass er die Daten speichert, wobei
die Steuereinheit (121) so konfiguriert ist, dass sie die Trenddaten in Zeitintervallen von einer Startzeit der Messung in der Feldvorrichtung (10) bis zu einem aktuellen Zeitpunkt im Speicher (122) speichert, um einen Kompressionsprozess für die im Speicher (122) gespeicherten Daten auszuführen, wenn eine Menge der im Speicher (122) gespeicherten Trenddaten eine obere Grenze einer Speicherkapazität des Speichers (122) erreicht, dass sie Daten eines gemessenen Trends auf der Grundlage der im Speicher (122) gespeicherten Trenddaten berechnet und einen vorhergesagten Trend berechnet, der eine zeitliche Änderung des Parameters ab dem aktuellen Zeitpunkt angibt,
die Feldvorrichtung weiter eine Ausgabeschnittstelle umfasst, die so konfiguriert ist, dass sie Informationen an einen Benutzer ausgibt, wobei die Steuereinheit so konfiguriert ist, dass sie den gemessenen Trend und den vorhergesagten Trend als Grafik auf der Ausgabeschnittstelle und/oder auf einer externen Vorrichtung, die mit der Feldvorrichtung kommunikationsfähig verbunden ist, anzeigt.

2. Feldvorrichtung (10) nach Anspruch 1, wobei die Steuereinheit so konfiguriert ist, dass sie den vorhergesagten Trend auf der Grundlage mindestens eines Vorhersagealgorithmus berechnet, der von einem Benutzer aus einer Vielzahl von Vorhersagealgorithmen ausgewählt wird.

3. Feldvorrichtung (10) nach Anspruch 1 oder 2, wobei die Steuereinheit (121) so konfiguriert ist, dass sie einen anfänglichen Änderungspunkt in dem gemessenen Trend berechnet und den vorhergesagten Trend auf der Grundlage des Vorhersagealgorithmus des mindestens einen Vorhersagealgorithmus, der von dem Benutzer ausgewählt wurde, berechnet, der einen Trend, der mit dem gemessenen Trend identisch ist, von einem Zeitpunkt, der dem Änderungspunkt entspricht, bis zum aktuellen Zeitpunkt als den vorhergesagten Trend herannimmt.

4. Feldvorrichtung (10) nach Anspruch 1, wobei die Steuereinheit (121) so konfiguriert ist, dass sie einen Bestimmungsgrad und eine Bestimmungszeit erfasst, die von einem Benutzer eingestellt werden, eine Zeit im vorhergesagten Trend, die dem Bestimmungsgrad entspricht, als Ankunftszeit berechnet und einen Benachrichtigungsprozess ausführt, um den Benutzer über ein Ergebnis der prädiktiven Diagnose zu benachrichtigen, nachdem bestimmt wurde, dass eine Zeit von der aktuellen Zeit bis zur Ankunftszeit gleich oder kleiner als die Bestimmungszeit ist.

5. Feldvorrichtung (10) nach Anspruch 4, wobei
die Steuereinheit (121) so konfiguriert ist, dass sie die berechnete Ankunftszeit und die Benachrichtigungsinformationen auf der Grundlage des Benachrichtigungsprozesses auf der Ausgabeschnittstelle (24) und/oder der externen Vorrichtung anzeigt.

6. Feldvorrichtung (10) nach einem der Ansprüche 1 bis 5, wobei die Steuereinheit (121) so konfiguriert ist, dass sie die Daten im Speicher (122) in Zyklen speichert und den Kompressionsprozess ausführt, indem sie erste Daten in einem Zyklus mit zweiten Daten in einem oder mehreren anderen Zyklen mittelt.

7. Messverfahren unter Verwendung einer Feldvorrichtung (10), wobei das Messverfahren Folgendes umfasst:
Detektieren einer physikalischen Größe und Ausgeben eines Detektionssignals;
Berechnen, auf der Grundlage des Detektionssignals, von Trenddaten zur Messung eines gemessenen Trends, der eine zeitliche Änderung eines Parameters angibt, der für die prädiktive Diagnose der Feldvorrichtung (10) verwendet wird:
Speichern der Trenddaten in einem Speicher (122) der Feldvorrichtung (10) in Zeitintervallen von einer Startzeit der Messung in der Feldvorrichtung (10) bis zu einer aktuellen Zeit;
Ausführen eines Kompressionsprozesses auf die im Speicher (122) gespeicherten trainierten Daten, wenn eine Menge der im Speicher (122) gespeicherten Trenddaten eine obere Grenze einer Speicherkapazität des Speichers (122) erreicht; und
Berechnen eines gemessenen Trends auf der Grundlage der im Speicher gespeicherten Trenddaten,
Berechnen eines vorhergesagten Trends, der eine zeitliche Änderung des Parameters ab dem aktuellen Zeitpunkt angibt, und
Anzeigen des gemessenen Trends und des vorhergesagten Trends als Grafik auf einer Ausgabeschnittstelle der Feldvorrichtung und/oder auf einer externen Vorrichtung, die mit der Feldvorrichtung kommunikativ verbunden ist.

8. Nichtflüchtiges computerlesbares Medium, das ein Programm speichert, das von einem oder mehreren Prozessoren ausführbar ist, um eine Feldvorrichtung (10) zu veranlassen, Funktionen nach Anspruch 7 auszuführen.

## Revendications

1. Dispositif de champ (10) comprenant :
un détecteur (11) configuré pour détecter une quantité physique et délivrer en sortie un signal de détection ;
un régulateur (121) configuré pour calculer, sur la base du signal de détection, des données de tendance pour mesurer une tendance mesurée indiquant un changement temporel dans un paramètre utilisé pour un diagnostic prédictif du dispositif de champ (10) ; et
une mémoire (122) configurée pour stocker les données, dans lequel
le régulateur (121) est configuré pour stocker les données de tendance dans la mémoire (122) à des intervalles de temps à partir d'une heure de départ d'une mesure dans le dispositif de champ (10) jusqu'à une heure actuelle, afin d'exécuter un processus de compression sur les données stockées dans la mémoire (122) lorsqu'une quantité des données de tendance stockées dans la mémoire (122) atteint une limite supérieure d'une capacité de stockage de la mémoire (122), pour calculer des données de tendance mesurées sur la base des données de tendance stockées dans la mémoire (122), et pour calculer une tendance prédite indiquant un changement temporel dans le paramètre à partir de l'heure actuelle,
le dispositif de champ comprenant en outre une interface de sortie configurée pour délivrer en sortie des informations à destination d'un utilisateur, dans lequel le régulateur est configuré pour afficher la tendance mesurée et la tendance prédite sous forme de graphique sur l'interface de sortie et/ou sur un dispositif externe relié de manière communicante au dispositif de champ.

2. Dispositif de champ (10) selon la revendication 1, dans lequel le régulateur est configuré pour calculer la tendance prédite sur la base d'au moins un algorithme prédictif sélectionné par un utilisateur parmi une pluralité d'algorithmes prédictifs.

3. Dispositif de champ (10) selon la revendication 1 ou 2, dans lequel le régulateur (121) est configuré pour calculer un point de changement initial dans la tendance mesurée et calculer la tendance prédite sur la base de l'algorithme prédictif de l'au moins un algorithme prédictif sélectionné par l'utilisateur qui prend une tendance identique à la tendance mesurée à partir d'une heure correspondant au point de changement par rapport à l'heure actuelle servant de tendance prédite.

4. Dispositif de champ (10) selon la revendication 1, dans lequel le régulateur (121) est configuré pour acquérir un niveau de détermination et une heure de détermination définis par un utilisateur, calculer une heure, dans la tendance prédite, correspondant au niveau de détermination servant d'heure d'arrivée, et exécuter un processus de notification afin de notifier à l'utilisateur un résultat de diagnostic prédictif lors d'une détermination qu'une heure à partir de l'heure actuelle jusqu'à l'heure d'arrivée est inférieure ou égale à l'heure de détermination.

5. Dispositif de champ (10) selon la revendication 4, dans lequel
le régulateur (121) est configuré pour afficher l'heure d'arrivée calculée et des informations de notification sur la base du processus de notification sur l'interface de sortie (24) et/ou le dispositif externe.

6. Dispositif de champ (10) selon l'une quelconque des revendications 1 à 5, dans lequel le régulateur (121) est configuré pour stocker les données dans la mémoire (122) dans des cycles et exécuter le processus de compression en faisant la moyenne de premières données dans un cycle avec des secondes données dans un ou plusieurs autres cycles.

7. Procédé de mesure utilisant un dispositif de champ (10), le procédé de mesure comprenant :
détecter une quantité physique et délivrer en sortie un signal de détection ;
calculer, sur la base du signal de détection, des données de tendance pour mesurer une tendance mesurée indiquant un changement temporel dans un paramètre utilisé pour un diagnostic prédictif du dispositif de champ (10) ;
stocker les données de tendance dans une mémoire (122) du dispositif de champ (10) à des intervalles de temps à partir d'une heure de départ d'une mesure dans le dispositif de champ (10) jusqu'à une heure actuelle ;
exécuter un processus de compression sur les données entraînées stockées dans la mémoire (122) lorsqu'une quantité des données de
tendance stockées dans la mémoire (122) atteignent une limite supérieure d'une capacité de stockage de la mémoire (122) ; et
calculer une tendance mesurée sur la base des données de tendance stockées dans la mémoire,
calculer une tendance prédite indiquant un changement temporel dans le paramètre à partir de l'heure actuelle, et
afficher la tendance mesurée et la tendance prédite sous forme de graphique sur une interface de sortie du dispositif de champ et/ou sur un dispositif externe relié de manière communicante au dispositif de champ.

8. Support non-transitoire lisible par ordinateur stockant un programme qui peut être exécuté par un ou plusieurs processeurs pour amener un dispositif de champ (10) à exécuter des fonctions selon la revendication 7.
